# EUROPÄISCHE PATENTSCHRIFT

(11) **EP 1 442 279 B1**
(45) Veröffentlichungstag und Bekanntmachung des Hinweises auf die Patenterteilung: **16.12.2009**
(21) Anmeldenummer: 02785315.9
(22) Anmeldetag: 26.10.2002
(51) Int. Cl.: G01N 1/06, G01N 1/31, G02B 21/34

(54) **VERFAHREN UND VORRICHTUNG ZUR ZUORDNUNG DER KENNZEICHNUNG VON OBJEKTTRAGERN FUR MIKROTOMIERTE GEWEBEPROBEN SOWIE ZUR VORBEREITUNG EINER SOLCHEN KENNZEICHNUNG**
METHOD AND DEVICES FOR THE CROSS-REFERENCING OF IDENTIFICATION OF OBJECT SUPPORTS FOR MICROTOMISED ANALYTICAL SAMPLES AND FOR THE GENERATION OF SAID IDENTIFICATION
PROCEDE ET DISPOSITIFS D'ASSOCIATION DE L'IDENTIFICATION DE PORTE-OBJETS CON US POUR DES ECHANTILLONS TISSULAIRES AYANT SUBI UNE MICROTOMIE ET DE PREPARATION D'UNE TELLE IDENTIFICATION

(30) Priorität: 08.11.2001 DE 10154843
(43) Veröffentlichungstag der Anmeldung: 04.08.2004
(73) Patentinhaber: Microm International GmbH, 69190 Walldorf (DE)
(72) Erfinder: HEID, Hans, L., 69245 Bammental (DE); NOVOA, José, 69118 Heidelberg (DE)
(74) Vertreter: Weber, Walter
(86) Internationale Anmeldenummer: PCT/EP2002/011983
(87) Internationale Veröffentlichungsnummer: WO 2003/040697

(56) Entgegenhaltungen:
- EP-A- 1 154 301
- WO-A-00/62035
- DE-A- 10 115 065
- GB-A- 2 235 163
- US-A- 4 276 253
- US-A- 5 561 556
- US-A- 5 746 855
- US-A- 5 854 075

## Beschreibung

Die Erfindung betrifft ein Verfahren zur Zuordnung der Kennzeichnung von Objektträgern für noch auf diese aufzubringende mikrotomierte Gewebeproben in Zuordnung zu einer Kennzeichnungsinformation, die einem Träger einer zugehörigen, noch nicht mikrotomierte Gewebeprobe zugeordnet ist.

Die Erfindung betrifft weiterhin eine Vorrichtung zur Durchführung eines solchen Verfahrens und eine Vorrichtung zur Vorbereitung der Durchführung eines solchen Verfahrens mit einer Kennzeichnung von Objektträgern für mikrotomierte Gewebeproben in Zuordnung zu einer Kennzeichnungsinformation, die einem Träger einer noch nicht mikrotomierten Gewebeprobe zugeordnet ist, wobei der Träger mit einem Informationsträger für die Zuordnung ausgestattet ist, und die Vorrichtung eine Einrichtung zur Erstellung der Kennzeichnungsinformation aufweist.

In der histologischen Technik werden im Routinebetrieb Gewebeproben von Patienten untersucht. Die verbreiteste Methode besteht darin, die Gewebeproben einer Paraffineinbettung zu unterziehen, sie dann mit Mikrotomen zu schneiden, in einem weiteren Prozeß anzufärben, mit einem Deckglas einzudecken und zur diagnostischen Auswertung einem Mikroskop zuzuführen. In fortschrittlichen Labors werden diese Patientenproben in automatisierter Weise in den einzelnen Schritten verarbeitet. Die Probeneinbettung erfolgt in den Schritten Fixierung, Entwässerung, Clearing mit Zwischenmedium und Einbettung in Paraffin in einem Einbettautomaten. Der nächste Schritt, das Herstellen eines Paraffinblockes wird auf einer Ausgießstation vorgenommen. Die Herstellung von mikroskopischen Dünnschnitten wird durch Schneiden des Paraffinblockes mit der eingebetteten Gewebeprobe auf einem Mikrotom erreicht. Das Anfärben und Kontrastieren des erzielten Dünnschnittes wird in mehreren Schritten in einem Färbeautomaten durchgeführt. Nach dem Eindecken des Dünnschnittes mit einem Deckglas auf einem Deckglaseindeckautomaten steht der aus der Patientenprobe hervorgegangene Dünnschnitt zur Auswertung unter einem Mikroskop bereit.

Eine Schwierigkeit besteht nun darin, die Gewebeproben durchgehend durch alle Prozeßschritte eindeutig identifizierbar zu halten, Verwechslungen auszuschließen und Probenverluste zu vermeiden. Diesbezüglich wurden bereits einige Fortschritte geleistet. So wird in einem modernen Prozeß die Gewebeprobe unmittelbar nach dem Zuschneiden in eine sogenannten Gewebekassette eingesetzt und mit einem Deckel verschlossen und gesichert. Fortschrittliche Gewebekassetten bieten neben guten Eigenschaften zur Flüssigkeitsdurchströmung auch ausgeprägte Merkmale zur Sicherung der Probe gegen Verlust und zur eindeutigen Identifizierung. Dazu besitzen fortschrittliche Kassetten eine beschriftbare oder bedruckbare Fläche ausreichender Größe und einer Oberflächenbeschaffenheit, die das Beschriften oder Bedrucken mit hinreichender Haftfähigkeit und Haltbarkeit während der folgenden Prozesse ermöglicht. Auf diese Weise bleibt die Probe während der Gewebeeinbettung eindeutig in der beschrifteten Kassette identifizierbar. Bei dem nächsten Schritt, dem Ausgießen eines Paraffinblockes um die Gewebeprobe herum, besteht weiterhin ausreichende Sicherheit vor Verwechslung von Proben, da die gleiche beschriftete Kassette als Träger des Paraffinblockes mit der Gewebeprobe dient und damit die Probe über den erstarrten Paraffinblock mit der beschrifteten Kassette verbunden ist.

Im wiederum nächste Schritt werden von der Gewebeprobe mikroskopische Dünnschnitte auf einem Mikrotom hergestellt. Dazu wird der Paraffinblock mit eingebetteter Gewebeprobe und verbundenem Kassettenträgerteil in die Probenhalterung eines Mikrotoms eingespannt und mikrotomiert. Dabei entstehen am Messerrücken des Mikrotommessers oder am Rücken von Einwegklingenhaltem Dünnschnitte, die über manuelle Verfahren mit Pinsel und Pinzette zur Schnittstreckung in ein Wasserbad mit warmem Wasser überführt werden. Sodann werden die gestreckten Dünnschnitte auf einen handelsüblichen Objektträger aus Glas aufgenommen. Der Objektträger muß nun auf seinem Beschriftungsfeld die gleiche zuordnende Information über die Gewebe enthalten, wie die, die in der Beschriftung der als Träger dienenden Gewebekassette niedergelegt ist. Zusätzlich ist es im allgemeinen üblich, daß von einer Gewebeprobe mehrere verwertbare Dünnschnitte angefertigt werden, die sich auf mehreren Objektträgern verteilt befinden. Dies bedeutet, daß mehrere Objektträger den gleichen Herkunftshinweis bezüglich der Gewebeprobe enthalten müssen.

Für die Beschriftung der Objektträger gibt es zwei Verfahren: Normalerweise werden die Objektträger handschriftlich beschriftet sobald die Dünnschnitte erzeugt sind. Dies bedeutet, daß die auf den Kassetten vorhandene Patientenprobeninformation durch handschriftliches Abschreiben auf einen oder mehrere Objektträger übertragen werden muß. Es ist nun leicht einzusehen, daß diese Methode die Gefahr von Übertragungsfehlern begünstigt und daher ein geringes Maß an Sicherheit gegen Verwechslung und Fehlerhaftigkeit bietet. Hinzu kommt, daß die handschriftlichen Informationen zu einem späteren Zeitpunkt von anderen Personen gelesen und weiterverarbeitet werden müssen.

Aus der US 4, 276, 253 ist es bekannt, auf der Probe eine Kennzeichnung anzubringen, die beim Schneiden für jede mikrotomierte Gewebeprobe erhalten bleibt, beziehungsweise vervielfältigt wird. Da hier die mikrotomierte Gewebeprobe schon beim Schneiden gekennzeichnet ist, gibt es kein zu lösendes Zuordnungsproblem. Dies erfordert natürlich eine aufwendige Probenkennzeichnung mit Markierungen aus länglichen Profilteilen, die mitgeschnitten werden, was eine Vergrößerung des zu schneidenden Parafinblocks erforderlich macht, sowie eine aufwendige Einbettung dieser Markierungen. Der erhebliche technische Aufwand macht eine Realisierung in der Praxis fraglich.

Weitere Schriften (WO 00/62035, US 5, 854, 075; US 5, 746, 855 und DE 100 10 140 A1) beschreiben automatische Prozesse, bei denen die Zuordnungsproblematik aufgrund ihrer maschinellen Durchführung nicht mehr auftritt. Eine solche völlige Automatisierung ist wegen der äußerst dünnen Schnitte problematisch und daher zumindest nur mit erheblichem technischem Aufwand realisierbar.

Es sind weiterhin auch ein Verfahren sowie Vorrichtungen der eingangs genannten Art mit manueller Zuordnung bekannt, bei denen zu Beginn des Probenvorbereitungsprozesses zum Zeitpunkt der Beschriftung der als Träger dienenden Gewebekassette bereits gleichzeitig einer oder mehrere Objektträger mit der gleichen Information vorbeschriftet werden. Dies mindert die Verwechslungsgefahr, besonders wenn die Beschriftung automatisiert mit einem gekoppelten Kassetten- und Objektträgerdrucksystem erfolgt. Ein Nachteil dieser Methode ist jedoch, daß die vorbeschrifteten Objektträger die Patientenprobe mit der Gewebekassette in den nachfolgenden Schritten nicht als miteinander verbundene Einheit begleiten können. Dies bedeutet, daß nach dem Mikrotomieren einer Gewebeprobe die dazu passenden vorbeschrifteten Objektträger herausgesucht und durch manuellen Vergleich der Beschriftungsinformation identifiziert werden müssen. Ein weiterer Nachteil ist der, daß zum Zeitpunkt der Beschriftung nicht sicher die Anzahl der benötigten Objektträger für die jeweilige Gewebeprobe vorbestimmt werden kann, da dies neben der Vorgabe durch den Arzt auch der Beurteilung der ausführenden Fachkraft beim Mikrotomieren obliegt. Eine Systematik einzuhalten, die eine Zuordnung der Objektträger zur Gewebeprobe des Patienten mit einem Wiederfinden der vorbeschrifteten Objektträger gewährleistet, ist in der Praxis schwer durchführbar, da die Proben in den einzelnen Vorbereitungsschritten mehrfach umgelagert werden müssen. Es sind daher umständliche Zwischenlagerungen der Objektträger und Lesevergleiche erforderlich, um erneut eine Zuordnung herzustellen.

Vorgefertigte Objektträger mit entsprechenden Kennzeichnungen sind nicht nur schwer zuzuordnen, sie müßten auch noch nachgefertigt werden oder in Überzahl vorhanden sein, wenn die Fachkraft, welche die Dünnschnitte herstellt, während dieses Vorgangs erkennt, daß mehr mikrotomierte Gewebeproben erforderlich sind als der Arzt vorgesehen hat. Diese Problematik verschärft sich, wenn die Kennzeichnung der Objektträger neben der Zuordnung zum Patienten weitere Hinweise für unterschiedliche Bearbeitung und Auswertung enthalten sollen.

Der Erfindung liegt daher die Aufgabe zugrunde, ein sicheres und effizienteres Kennzeichnungsverfahren für Objektträger sowie Vorrichtungen verfügbar zu machen, welche auf einfache Weise die beschriebene Zuordnungsproblematik in der histologischen Technik bei manueller Zuordnung entschärfen.

Die Aufgabe wird bezüglich eines Verfahrens der eingangs genannten Art dadurch gelöst, daß die zum Träger gehörende Kennzeichnungsinformation während der Anordnung desselben im Mikrotom automatisch erfaßt und eine dieser zuzuordnende Kennzeichnung auf mindestens einen Objektträger automatisch übertragen wird und daß ausschließlich dieser mit der Kennzeichnung versehene Objektträger zu dem Zeitpunkt, an dem eine mikrotomierte Gewebeprobe auf einen Objektträger gebracht werden muß, am Arbeitsplatz des Mikrotoms für die manuelle Aufbringung einer mikrotomierten Gewebeprobe der am Mikrotom arbeitenden Fachkraft dargeboten wird.

Bezüglich der Vorrichtung zur Durchführung des Verfahrens wird die Aufgabe dadurch gelöst, daß diese eine Erfassungseinrichtung zur Erfassung der Kennzeichnungsinformation während der Träger mit einer nicht mikrotomierten Gewebeprobe in der Halteeinrichtung eines Mikrotoms angeordnet ist, aufweist, sowie eine Kennzeichnungseinrichtung zur Ausstattung eines Objektträgers mit einer Kennzeichnung, eine Übertragungseinrichtung zur Informationsübertragung zwischen der Erfassungseinrichtung und der Kennzeichnungseinrichtung und eine am Arbeitsplatz des Mikrotoms installierte Ausgabeeinrichtung für die Entnahme von mit einer Kennzeichnung versehenen Objektträgern durch die am Mikrotom arbeitende Fachkraft zum manuellen Aufbringen der mikrotomierten Gewebeprobe.

Durch das erfindungsgemäße Verfahren wird der Fachkraft, die die mikrotomierten Proben - also die Dünnschnitte - herstellt, die Arbeit der Zuordnung dieser Proben zu der nicht mikrotomierten Gewebeprobe völlig abgenommen. Nicht mikrotomierte Gewebeproben sind in diesem Sinn auch solche Gewebeproben, von denen die ersten Dünnschnitte bereits abgeschnitten sind. Vor allem ist es durch die automatisierte Erfassung möglich, diese zu erfassen, wenn sich der Träger mit der Gewebeprobe im Mikrotom befindet, was bei einer Erfassung durch das menschliche Auge wegen der weitgehendsten Unlesbarkeit einer Beschriftung aufgrund der engen Raumverhältnisse zumindest nicht auf zuverlässige Art und Weise möglich ist. Eine Erfassung außerhalb des Mikrotoms vermeidet die Erfindung, da nicht garantiert ist, daß die erfaßte Gewebeprobe auch eingespannt wird. Fehler bei der Kennzeichnung der Objektträger sind durch die automatische Erfassung der Kennzeichnungsinformation während der Einspannung der Träger sowie durch die automatische Übertragung und Kennzeichnung ausgeschlossen. Die Übertragung kann elektrisch, optisch, durch Infrarot, Funk oder in anderer Weise erfolgen.

Die Zuverlässigkeit wird weiter dadurch erzielt, daß der Fachkraft immer nur ein solcher Objektträger zur Verfügung steht, der bezüglich seines Herkunftshinweises mit der mikrotomierten Gewebeprobe übereinstimmt, da in dem Moment, indem die Fachkraft die mikrotomierte Gewebeprobe auf einen Objektträger bringen muß, nur der Objektträger mit der richtigen Kennzeichnung dargeboten wird. Es entfällt also jegliche menschliche Zuordnungsarbeit. Mit dem Entfallen der Zuordnungsarbeit entfällt aber auch die Möglichkeit, daß bei einer solchen Fehler unterlaufen können. Selbst wenn die Fachkraft das Mikrotomieren einer Probe unterbricht, um zwischendurch eine andere Probe zu bearbeiten, kann es keine Zuordnungsprobleme geben, da immer nur Objektträger dargeboten werden, die eine Kennzeichnung tragen, die der Kennzeichnungsinformation der im Mikrotom befindlichen Gewebeprobe zugeordnet ist. Kennzeichnungsinformation und Kennzeichnung können dabei materiell oder elektronisch mit dem Träger beziehungsweise dem Objektträger verbunden sein. Im letzteren Fall dient ein materiell zugeordneter Identifikationscode der elektronischen Zuordnung. Die letztgenannte Lösung ist bei großen Datenmengen oder dann zu bevorzugen, wenn eine körperliche Informationsverknüpfung mit umfangreichen Daten wegen der vorzunehmenden Bearbeitungen problematisch ist.

Die erfindungsgemäße Vorrichtung stellt die zur Durchführung des erfindungsgemäßen Verfahrens erforderlichen Einrichtungen bereit. Diese Einrichtungen sorgen dafür, daß die Erstellung und Darbietung der richtig gekennzeichneten Objektträger - und nur dieser! - automatisch und "just in time" sowie am Ort des Bedarfs und in der gewünschten Menge erfolgen kann.

Verfahren und Vorrichtung betreffen selbstverständlich nicht nur die eingangs genannte Art, mikrotomierte Gewebeproben herzustellen, da das Problem unabhängig davon ist, wie die Gewebeprobe behandelt und geschnitten wird. Es kann sich somit um ein Mikrotom handeln, bei dem das Messer bewegt wird oder bei dem das Messer feststeht und die Probe bewegt wird. Es kann eines dieser Elemente auf einem Schlitten oder einer rotierenden Scheibe bewegt werden und von Hand oder maschinell angetrieben sein. Selbstverständlich kann statt des Messers auch ein Laserstrahl oder ein anderes Verfahren zur Herstellung von Gewebeschnitten eingesetzt werden. Es kann sich nicht nur um menschliche sondern auch tierische oder pflanzliche Gewebeschnitte handeln.

Die Vorrichtung zur Vorbereitung einer Kennzeichnung von Objektträgern der eingangs genannten Art sieht vor, daß diese Einrichtung sowie der Informationsträger und ein dem Träger zugeordneter Datenträger für die Erstellung einer Kennzeichnungsinformation ausgebildet sind, die neben der Herkunftszuordnung der Gewebeprobe einen Informationsverteilungsschlüssel und Informationen beinhalten, die einer individuellen Informationsausstattung der Kennzeichnung von mehreren Objektträgern mit mikrotomierten Gewebeproben dienen, die von derselben noch nicht mikrotomierten Gewebeprobe strammen.

Durch diese Vorrichtung wird die Kennzeichnung der Objektträger vorbereitet, indem die erforderlichen Informationen als Kennzeicheninformation derart festgehalten werden, daß sie mechanisch oder elektronisch mit dem Träger der Gewebeprobe derart verknüpft sind, daß auf dieser Grundlage das oben genannte Verfahren mittels der oben genannten Vorrichtung durchführbar ist, wobei diese Vorrichtung zur Vorbereitung der Kennzeichnung Anwendungsfälle betreffen, in denen die Kennzeichnungsinformation Zuordnungsinformationen beinhalten, die individuelle Informationsausstattungen für einzelne Objektträger mit von derselben Gewebeprobe stammenden Dünnschnitten vorsehen. Auf diese Weise wird nicht nur die richtige Zuordnung der Kennzeichnung von Objektträgern für mikrotomierte Gewebeproben zu den entsprechenden noch nicht mikrotomierten Gewebeproben erzielt, es wird außerdem gewährleistet, daß eine gewünschte Anzahl von Gewebeschnitten auf Objektträgern mit den gewünschten Zusatzinformationen erstellt wird.

Im folgenden werden Weiterbildungen des Verfahrens und der erwähnten Vorrichtungen, sowie die erfindungsgemäße Vorrichtung zur Bearbeitung beschrieben.

Das erfindungsgemäße Verfahren wird zweckmäßigerweise derart weitergebildet, daß mit jeder Herstellung einer mikrotomierten Gewebeprobe die Kennzeichnungsinformation erfaßt und ein Objektträger mit einer Kennzeichnung hergestellt und dargeboten wird. Damit ist gewährleistet, daß für jede mikrotomierte Gewebeprobe auch ein Objektträger dargeboten wird. Dabei kann natürlich auch vorgesehen sein, daß die Herstellung des Objektträgers nur dann erfolgt, wenn die das Mikrotom bedienende Fachkraft die mikrotomierte Gewebeprobe als brauchbar erachtet und die Herstellung des gekennzeichneten Objektträgers freigibt.

Im einfachsten Fall enthält die Kennzeichnung des mindestens einen Objektträgers in identischer Weise die Kennzeichnungsinformation die der nicht mikrotomierten Gewebeprobe zugeordnet ist. In diesem Fall ist meist lediglich ein Herkunftshinweis auf die nicht mikrotomierte Gewebeprobe oder auf den Patienten, von dem diese stammt, enthalten. Es kann jedoch auch vorgesehen sein, daß die Kennzeichnungsinformation neben der Herkunftszuordnung der Gewebeprobe weitere Informationen enthält. Solche Informationen können die Vorbehandlung der Gewebeprobe, das Datum oder den Ort der Entnahme beziehungsweise das Organ der Entnahme betreffen. Weitere Informationnen sind denkbar, Beispiele hierfür werden noch genannt.

Sollen die mikrotomierten Gewebeproben unterschiedlich behandelt oder unterschiedlich ausgewertet oder in ihrer Reihenfolge festgelegt werden, so ist es zweckmäßig, wenn die Kennzeichnung der Objektträger aufgrund eines Informationsverteilungsschlüssels neben der Herkunftszuordnung mit weiteren Informationen geladen wird, welche die von derselben nicht mikrotomierten Gewebeprobe stammenden einzelnen mikrotomierten Gewebeproben auf den Objektträgern individualisieren. Dadurch kann die Weiterbehandlung und Auswertung aufgrund der Kennzeichnung erfolgen und es ist nicht erforderlich, den Objektträgern gesondert derartige Informationen beizufügen. Auch dadurch wird die Zuordnungsproblematik in der histologischen Technik entschärft. Zweckmäßigerweise sind der Informationsverarbeitungsschlüssel und die weiteren Informationen in der Kennzeichnungsinformation enthalten und dadurch körperlich oder elektronisch mit dem Träger und somit der nicht mikrotomierten Gewebeprobe verbunden.

Auch bezüglich der Vorrichtung zur Durchführung des vorgenannten Verfahrens sind verschiedene Ausgestaltungen möglich. So kann die Erfassungseinrichtung derart ausgebildet sein, daß sie nach dem Positionieren des Trägers im Mikrotom die Kennzeichnungsinformation erfaßt und daß sie die Kennzeichnung mindestens eines Objektträgers veranlaßt. Die Erfassungseinrichtung kann jedoch auch derart ausgebildet sein, daß es bei der Herstellung einer mikrotomierten Gewebeprobe zu einer Erfassung der Kennzeichnungsinformation und zu einem Befehl an die Kennzeichnungseinrichtung zur Vornahme der Kennzeichnung eines Objektträgers kommt. Auch eine laufende Erfassung der Kennzeichnungsinformation und eine Vornahme der Kennzeichnung von Objektträgern bei Bedarf, zum Beispiel durch einen Knopfdruck ist möglich.

Kennzeichnungsinformation und Kennzeichnung können selbstverständlich auf die unterschiedlichste Weise ausgebildet sein, es kann sich um eine durch den Menschen lesbare Beschriftung oder eine maschinell auswertbare Beschriftung, wie ein Strichcode, jedoch auch um ein magnetisches oder elektronisches Speichermedium jeglicher Art handeln. Dabei kann vorgesehen sein, daß sich die Kennzeichnungsinformation oder Kennzeichnung auf einem separaten Datenträger befindet, die einem Träger oder einem Objektträger mittels eines Identifikationscodes zuordenbar ist. Dann muß selbstverständlich gewährleistet sein, daß nur die richtige Zuordnung zulässig ist. Diese Lösung ist dann zweckmäßig, wenn umfangreichere Daten zugeordnet werden sollen, was eher bei der Kennzeichnungsinformation der Fall ist. Oft ist es jedoch ausreichend und auch eine einfache Ausgestaltung, wenn die Kennzeichnungsinformation auf einem Informationsträger geladen ist, der mit dem Träger körperlich verbunden ist.

Um zu verhindern, daß in der Ausgabeeinrichtung von der vorder bearbeiteten Gewebeprobe ein Objektträger zurückbleibt und dadurch ein Zuordnungsfehler unterläuft, ist es zweckmäßig, wenn die Ausgabeeinrichtung derart ausgestaltet ist, daß sie die Darbietung eines mit einer Kennzeichnung versehenen Objektträgers abbricht, wenn ein neuer Träger im Mikrotom plaziert wird. Zu diesem Zweck kann die Ausgabeeinrichtung den verbliebenen. Objektträger verwerfen oder zurückziehen, um ihn mit einer neuen Information zu beschreiben.

Geht die Funktion der Vorrichtung über das reine Kopieren einer Herkunftszuordnung hinaus, so ist es zweckmäßig, wenn sie eine Steuerung zur Durchführung der Kennzeichnung der Objektträger aufweist. Ist eine solche Steuerung vorhanden, so ist es weiterhin zweckmäßig, wenn die Vorrichtung eine Befehlseingabe für die Bestimmung der Art und/oder der Anzahl der Kennzeichnungen aufweist. Bei dieser Befehlseingabe kann es sich um eine manuelle Befehlseingabe handeln oder es kann eine Befehlseingabe mittels der Kennzeichnungsinformation vorgenommen werden. Auch eine externe Befehlseingabe durch eine mit einem Computer verbundene Datenleitung oder eine Befehlseingabe mittels eines Datenträgers ist denkbar.

Selbstverständlich sollte die Vorrichtung, am besten die Kennzeichnungseinrichtung, ein Depot für noch ungekennzeichnete Objektträger aufweisen, das ausreichend groß ist, um immer in ausreichendem Maß gekennzeichnete Objektträger herstellen zu können, ohne daß ein ständiges Nachladen erforderlich ist.

Die Steuerung kann derart ausgebildet sein, daß die Anzahl der mit einer Kennzeichnung zu versehenden Objektträger vorgebbar ist. Dies entspricht der Tatsache, daß in der Regel der Arzt vorgibt, wie viele mikrotomierte Gewebeproben auf Objektträgern erforderlich sind. Weiterhin sollte jedoch die Steuerung derart ausgebildet sein, daß die Anzahl der mit einer Kennzeichnung zu versehenden Objektträger manuell erhöht werden kann. Dies entspricht der Tatsache, daß die am Mikrotom arbeitende Fachkraft oft erkennt, daß mehr als die vorgegebenen Anzahl von Objektträgern erforderlich ist, sei es, daß die Qualität der Gewebeprobe zweifelhaft ist, oder daß diese Fachkraft bereits an dem Dünnschnitt erkennt, daß Besonderheiten mehr Dünnschnitte für eine Diagnose erforderlich machen.

Wie bereits oben zur Befehlseingabe ausgeführt, kann die Steuerung auf verschiedenste Weise ihre Informationen erhalten. Eine zweckmäßige Ausgestaltung mit geringsten Fehlermöglichkeiten, sieht jedoch vor, daß die Steuerung derart ausgebildet ist, daß sie die Informationen für die Vornahme der Anzahl und/oder Art der Kennzeichnung einer diese Informationen enthaltenden Kennzeichnungsinformation entnimmt.

Insbesondere, wenn eine Befehlseingabe und/oder eine Steuerung bei der Vorrichtung vorhanden sind, ist es zweckmäßig, wenn sie weiterhin eine Anzeigeeinrichtung zur Darstellung von Informationen und/oder Arbeitsschritten aufweist. Dadurch wird die mikrotomierende Fachkraft darüber informiert, wie viele Dünnschnitte in welcher Weise und mit welchen Informationen hergestellt werden. Dies ermöglicht eine zusätzliche Kontrolle und ergänzende Eingaben.

Eine praktische Ausbildung der Vorrichtung sieht vor, daß die Erfassungseinrichtung, die Träger und die Halteeinrichtung derart ausgebildet und angeordnet sind, daß die Erfassungseinrichtung die Kennzeichnungsinformation von einem Lesefeld des Trägers ablesen kann. Zu diesem Zweck kann vorgesehen sein, daß Träger und Halteeinrichtung derart ausgebildet und aufeinander abgestimmt sind, daß sich die Spannbacken einer als Spanneinrichtung ausgebildeten Halteeinrichtung außerhalb des Lesefelds eines eingespannten Trägers befinden. Dies kann durch eine entsprechende Anordnung und Ausgestaltung der Erfassungseinrichtung oder auch durch eine Modifikation der Spannbacken gegenüber den bisherigen Einspannvorrichtungen erzielt werden, wobei das Ziel darin besteht, am Lesefeld genügend Raum für eine Erfassungseinrichtung zur Verfügung zu haben. Der Begriff "Lesefeld" ist natürlich dahingehend zu verstehen, daß nicht nur eine optische sondern auch eine magnetisch oder elektronisch hinterlegte Information gelesen werden kann.

Auch die Vorrichtung zur Vorbereitung einer Kennzeichnung von Objektträgern, durch Erstellung von Kennzeichnungsinformationen, die den Trägem zugeordnet werden, kann auf unterschiedlichste Weise ausgestaltet sein. So ist es möglich, daß die Informationsträger auf unterschiedlichste Weise ausgebildet sind, und auch daß die Einrichtungen zur Erstellung der Kennzeichnungsinformation entsprechend verschieden ausgebildet sind. Beispielsweise kann vorgesehen sein, daß die Einrichtung zur Erstellung der Kennzeichnungsinformation derart ausgebildet ist, daß sie letztere auf einem separaten Datenträger lädt und diesen oder einen bestimmten Dateninhalt eines Datenträgers sowie den Informationsträger des Trägers der Gewebeprobe mit einem Identifikationscode zur gegenseitigen Zuordnung versieht. Diese Ausgestaltung ist vor allen Dingen dann zweckmäßig, wenn den Gewebeproben in nicht mikrotomierter und/oder mikrotomierter Form sehr viele Daten zugeordnet werden müssen und der Träger daher nicht ausreicht, um ein entsprechend großes Speichermedium an ihm zu befestigen oder wenn die noch vorzunehmende Bearbeitung einer Probe ein solches Speichermedium nicht zuläßt. Eine alternative Ausgestaltung sieht vor, daß die Einrichtung und der Informationsträger derart ausgebildet sind, daß letzterer mit der gesamten Kennzeichnungsinformation ladbar ist.

Mit Hilfe des Informationsverteilungsschlüssels können die Objektträger verschiedene Informationsausstattungen erhalten. Beispielsweise ist es möglich, daß die Objektträger unterschiedliche Bearbeitungsanweisungen für die mikrotomierten Gewebeproben erhalten, oder daß individuelle Auswertungshinweise für die mikrotomierten Gewebeproben in den Informationsausstattungen der Objektträger enthalten sind.

Letztlich kann die vorgenannte Vorrichtung gemeinsam mit dem Verfahren und der Vorrichtung zur Durchführung des Verfahrens auch für eine weitgehende Automatisierung eingesetzt werden. Zu diesem Zweck ist es von Vorteil, wenn die vorgenannten Informationsausstattungen als maschinell erfaß- und umsetzbare Gerätesteuerungsanweisungen ausgestaltet sind.

Um diese umzusetzen, ist eine Vorrichtung zur Bearbeitung von auf Objektträgern angeordneten mikrotomierten Gewebeproben zweckmäßig, welche, eine Einrichtung zur Erfassung von Kennzeichnungen von Objektträgern und eine Gerätesteuerung aufweist, die derart ausgebildet ist, daß sie die Gerätesteuerungsanweisungen umsetzt, welche die Kennzeichnungen der Objektträger beinhalten. Auch eine solche Automatisierung, die auf den vorgehenden erfindungsgemäßen Maßnahme aufbaut, dient einer Entschärfung der Zuordnungsproblematik, in diesem Fall bezüglich der weiteren Bearbeitung.

Selbstverständlich sind weitere Ausgestaltungen und Weiterbildungen denkbar, insbesondere lassen viele Verfahrensmerkmale als Vorrichtungsmerkmale und umgekehrt realisieren.

Die Erfindung wird nachfolgend anhand von Ausführungsbeispielen für die Vorrichtung erläutert. Es zeigen
- **Fig. 1**: ein Ausführungsbeispiel einer Vorrichtung zur Durchführung des Verfahrens,
- **Fig. 2a**: ein Ausführungsbeispiel eines als Kassette ausgebildeten Trägers mit Lesefeld,
- **Fig. 2b**: einen Kassettendeckel für die vorgenannte Kassette,
- **Fig. 2c**: eine Kassette mit einer anders ausgebildeten Kennzeichnungsinformation,
- **Fig. 3a**: eine bekannte Halteeinrichtung,
- **Fig. 3b**: eine für die Anordnung einer Erfassungseinrichtung ausgebildete Halteeinrichtung und
- **Fig. 4**: ein Ausführungsbeispiel einer Vorrichtung zur Erstellung einer Kennzeichnungsinformation.

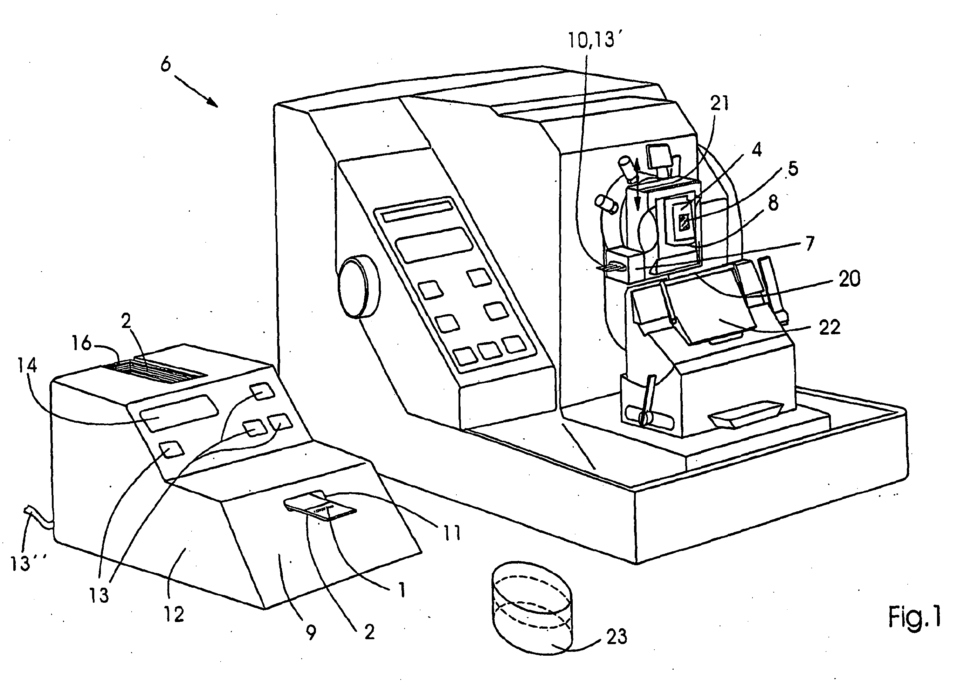

**Fig. 1** zeigt ein Ausführungsbeispiel einer erfindungsgemäßen Vorrichtung zur Durchführung des erfindungsgemäßen Verfahrens. Dargestellt ist ein Mikrotom 6, in dessen Halteeinrichtung 8 auf einem verfahrbaren Schlitten ein Träger 4 eingespannt ist, der eine noch nicht mikrotomierte Gewebeprobe 5 trägt. Diese noch nicht mikrotomierte Gewebeprobe 5 wird in Richtung eines Doppelpfeils 21 verschoben, um mittels des Messers 20 des Mikrotoms 6 Dünnschnitte herzustellen. Die Schlittenführung und Zustellung zur Erzielung der Schnittdicke sind wesentliche Funktionen des Mikrotoms 6. Diese Dünnschnitte sind die mikrotomierten Gewebeproben, die sich auf der Ablage 22 ablegen und in eingangs beschriebener Art und Weise abgenommen werden, um sie einem Wasserbad 23 zuzuführen. Durch das warme Wasserbad 23 werden die mikrotomierten Gewebeproben, also die Dünnschnitte, geglättet und können dann auf einen Objektträger 2 aufgebracht werden.

Die Erfindung löst das Problem, daß die Kennzeichnung 1 der Objektträger 2 der Kennzeichnungsinformation 3 der noch nicht mikrotomierten Gewebeprobe 5 sicher und effizient zugeordnet wird. Dazu ist vorgesehen, daß am Mikrotom 6 eine Erfassungseinrichtung 7 angeordnet ist, die die Kennzeichnungsinformation 3 des Trägers 4 erfaßt und mittels einer Übertragungseinrichtung 10 einer Kennzeichnungseinrichtung 9 für Objektträger 2 weitervermittelt. Entsprechend der Kennzeichnungsinformation 3 erstellt diese Kennzeichnungseinrichtung 9 eine Kennzeichnung 1 eines Objektträgers 2, der dann an der Ausgabeeinrichtung 11 entnommen werden kann, um die mikrotomierte Gewebeprobe aus dem Wasserbad 23 aufzunehmen. Auf diese Weise ist die Zuordnung der Kennzeichnung 1 auf dem Objektträger 2 zu der Kennzeichnungsinformation 3 des Trägers 4 immer gewährleistet. Wesentlich ist, daß die Erstellung der Kennzeichnung 1 des Objektträgers 2 immer an die Kennzeichnungsinformation 3 des in der Halteeinrichtung 8 befindlichen Trägers 4 gebunden ist. Nach Entfernung des Trägers 4 darf also keine ihm zuzuordnende Kennzeichnung 1 mehr erstellbar sein. Mit dem Einspannen eines neuen Trägers 4 oder bereits der Entfernung des alten Trägers 4 müssen noch gekennzeichnete Objektträger 2 aus der Ausgabeeinrichtung 11 und natürlich aus dem Bereich des Arbeitsplatzes des Mikrotoms 6 entfernt werden, damit sie nicht verbleiben und dadurch möglicherweise einer mikrotomierten Gewebeprobe 5 zugeordnet werden, die nicht mit deren Kennzeichnungsinformation 3 übereinstimmt. Vorzugsweise ist dazu die Kennzeichnungseinrichtung 9 derart ausgebildet, daß sie nicht gebrauchte gekennzeichnete Objektträger 2 vor der Bearbeitung einer neuen, noch nicht mikrotomierten Gewebeprobe 5 zurückzieht.

Das Ausführungsbeispiel weist eine Übertragungseinrichtung 10 auf, die beispielsweise als Infrarotübertragung ausgestaltet ist. Ein entsprechender Empfänger ist an der Kennzeichnungseinrichtung 9 angeordnet. Durch das Bezugszeichen 13' ist angedeutet, daß die erfaßte Kennzeichnungsinformation 3 auch eine Befehlseingabe 13' beinhalten kann, welche ebenfalls mit der Übertragungseinrichtung 10 übermittelt wird. Eine derartige Befehlseingabe dient individualisierten Kennzeichnungen 1 von Objektträgern 2 in der Art und Weise, wie dies bereits oben beschrieben wurde. Alternativ ist natürlich auch eine manuelle Befehlseingabe 13 oder eine externe Befehlseingabe 13" denkbar, letztere kann beispielsweise mit einem Computer verbunden sein. Mittels einer Anzeigeeinrichtung 14 kann die bedienende Person über Anzahl und Art der Kennzeichnungen 1 oder weiteres informiert werden.

Die Kennzeichnungseinrichtung 9 ist hier in einem Gehäuse untergebracht, das auch eine Steuerung 12 enthält, welche beispielsweise dazu dient, Befehle zu verarbeiten oder Kennzeichnungsinformationen 3 in individualisierte Kennzeichnungen 1 von Objektträgern 2 umzusetzen. Auch diesbezüglich wird auf die obige Beschreibung verwiesen. Weiterhin weist die Kennzeichnungseinrichtung 9 ein Depot 16 auf, in das nicht gekennzeichnete Objektträger 2 eingefüllt werden können, so daß ein ausreichender Vorrat vorhanden ist.

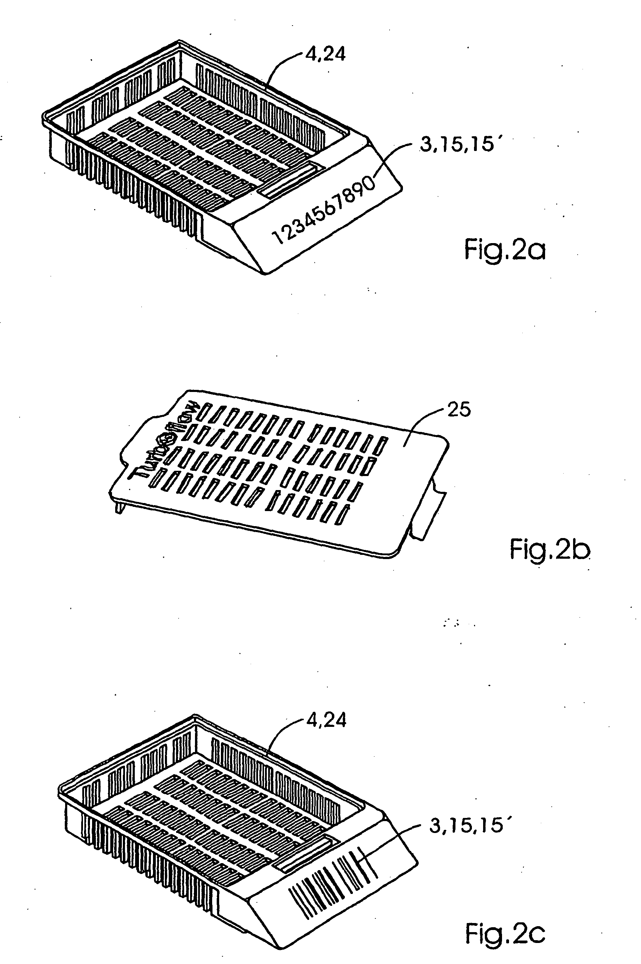

**Fig. 2a** zeigt ein Ausführungsbeispiel eines als Kassette 24 ausgebildeten Trägers 4 mit einer Kennzeichnungsinformation 3, welche auf einem als Lesefeld 15' ausgebildeten Informationsträger 15 aufgebracht ist. Selbstverständlich sind auch andere Ausgestaltungen eines Informationsträgers 15 denkbar, wie elektromagnetische Streifen, ein elektronischer Speicher oder ein Strichcode, wie er in **Fig. 2c** dargestellt ist. In die Kassetten 24 werden die nicht mikrotomierten Gewebeproben 5 hineingegeben und dann mit einem in **Fig. 2b** dargestellten Deckel 25 verschlossen. Danach erfolgt die Behandlung der Gewebeproben, wie sie eingangs geschildert wurde. Zum Schluß wird die nicht mikrotomierte Gewebeprobe 5 mit Paraffin durchtränkt und als Paraffinblock mit der Kassette 24 verbunden, so daß diese als Träger 4 für die noch nicht mikrotomierte Gewebeprobe 5 dient.

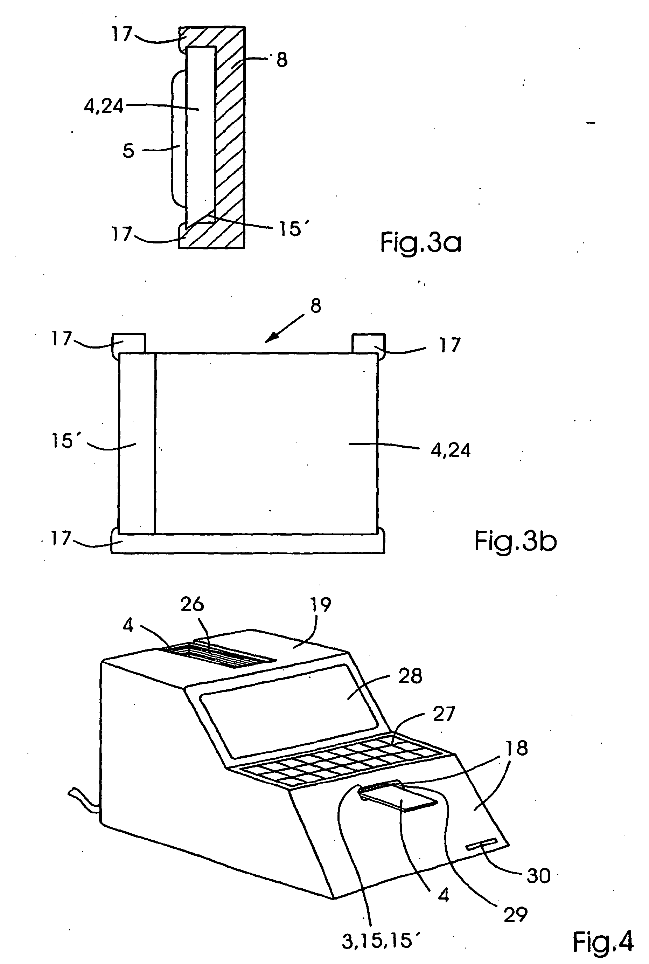

**Fig. 3a** zeigt eine Halteeinrichtung 8 vorbekannter Art, die den eben beschriebenen Träger 4, also die Kassette 24, verbunden mit der noch nicht mikrotomierten Gewebeprobe 5 am Mikrotom 6 zur Herstellung der Dünnschnitte plaziert. Bei der Halteeinrichtung 8 bekannter Art sind Spannbacken 17 vorgesehen, welche die Kassette 24 am Lesefeld 15' und an der gegenüberliegenden Seite halten. Dadurch ist es schwierig eine Erfassungseinrichtung 7 unterzubringen. Dem kann beispielsweise dadurch abgeholfen werden, daß die Halteeinrichtung 8 entsprechend der Darstellung in Fig. 3b ausgebildet wird, daß also die Spannbacken 17 die Kassette 24 an den Längsseiten halten. Dies ist selbstverständlich nur ein Vorschlag, viele weitere Möglichkeiten zur Unterbringung der Erfassungseinrichtung 7 sind denkbar.

**Fig. 4** zeigt ein Ausführungsbeispiel einer Vorrichtung 18 zur Vorbereitung einer Kennzeichnung 1 von Objektträgern 2. Diese Vorbereitung wird dadurch vorgenommen, daß Träger 4 mit einer Kennzeichnungsinformation 3 versehen werden können, welche dann durch die Steuerung 12 der Kennzeichnungseinrichtung 9 in entsprechende Kennzeichnungen 1 von Objektträgern 2 umgesetzt werden können, wie dies bereits ausführlich beschrieben wurde. Die Vorrichtung 18 besteht daher aus einer Einrichtung 19 zur Erstellung einer Kennzeichnungsinformation 3 sowie aus den Trägern 4 mit Informationsträgern 15, 15'. Die Einrichtung 19 zur Erstellung einer solchen Kennzeichnungsinformation 3 verfügt über eine Eingabe 27, welche beispielsweise als Tastatur ausgebildet ist, sowie über eine Anzeige 28, beispielsweise ein Bildschirm. Es können also sämtliche Daten eingegeben werden, um den Informationsträger 15 eines Trägers 4 mit der entsprechenden Kennzeichnungsinformation 3 zu laden. Alternativ kann dieser Vorgang natürlich auch dadurch ausgeführt werden, daß eine Datenträgeraufnahme 30 vorgesehen ist, in die ein Datenträger, beispielsweise eine Diskette eingegeben wird, welche die entsprechende Kennzeichnungsinformation 3 beinhaltet. Dann erstellt die Einrichtung 19 entsprechend dieser Daten die Kennzeichnungsinformation 3 auf dem Träger 4. Auch eine Datenleitung zu einem Rechner wäre zur Erstellung der Kennzeichnungsinformation 3 denkbar. Um ein zügiges Arbeiten zu ermöglichen, ist ein Depot 26 für Träger 4 ohne Kennzeichnungsinformation 3 vorgesehen.

Selbstverständlich muß an dieser Vorrichtung 18 eine besondere Vorkehrung dafür getroffen werden, daß der Träger 4 mit der Kennzeichnungsinformation 3 mit der richtigen Gewebeprobe 5 zusammengeführt wird. Aufgrund der dargestellten Erfindung ist jedoch dies der letzte kritische Bereich, der als erstmalige Zuordnung einer Gewebeprobe 5 nach deren Entnahme aus dem Körper nicht beseitigt werden kann. Jegliche weitere Bearbeitung und Auswertung der Gewebeprobe 5, ist jedoch durch die Erfindung gegen jegliche Zuordnungsverwechslung gesichert.

Die Figuren zeigen selbstverständlich nur einen geringen Teil der Möglichkeiten der Ausgestaltung der erfindungsgemäßen Vorrichtung. Sie beschränkt sich auf die Anwendung an einem marktüblichen Mikrotom 6. Dieses kann auch anders ausgestaltet sein, die Kennzeichnungseinhchtung 9 kann an dieses auch angebaut sein, letztere kann anders ausgestaltet sein.

### Bezugszeichenliste

- 1: Kennzeichnung eines Objektträgers für mikrotomierte Gewebeproben
- 2: Objektträger
- 3: Kennzeichnungsinformation, einem Träger für noch nicht mikrotomierte Gewebeproben zugeordnet
- 4: Träger
- 5: noch nicht mikrotomierte Gewebeprobe
- 6: Mikrotom
- 7: Erfassungseinrichtung
- 8: Halteeinrichtung
- 9: Kennzeichnungseinrichtung
- 10: Übertragungseinrichtung
- 11: Ausgabeeinrichtung
- 12: Steuerung
- 13, 13', 13": Befehlseingabe
- 13: manuelle Befehlseingabe
- 13': Befehlseingabe mittels der Kennzeichnungsinformation
- 13": externe Befehlseingabe oder Befehlssignale mittels eines Datenträgers
- 14: Anzeigeeinrichtung
- 15: Informationsträger
- 15': Lesefeld
- 16: Depot für ungekennzeichnete Objektträger
- 17: Spannbacken
- 18: Vorrichtung zur Vorbereitung einer Kennzeichnung von Objektträgern
- 19: Einrichtung zur Erstellung einer Kennzeichnungsinformation
- 20: Messer des Mikrotoms
- 21: Doppelpfeil: Verschiebung der Halteeinrichtung zur Vornahme der Dünnschnitte
- 22: Ablage der Dünnschnitte
- 23: Wasserbad
- 24: Kassette
- 25: Kassettendeckel
- 26: Depot für Träger ohne Kennzeichnungsinformation
- 27: Eingabe (Tastatur)
- 28: Anzeige (Bildschirm)
- 29: Ausgabe für Träger mit Kennzeichnungsinformation
- 30: Datenträgeraufnahme

## Patentansprüche

1. Verfahren zur Zuordnung der Kennzeichnung (1) von Objektträgern (2) für noch auf diese aufzubringende mikrotomierte Gewebeproben zu einer Kennzeichnungsinformation (3), die einem Träger (4) einer zugehörigen, noch nicht mikrotomierten Gewebeprobe (5) zugeordnet ist,
**dadurch gekennzeichnet,**
**daß** die zum Träger (4) gehörende Kennzeichnungsinformation (3) während der Anordnung desselben im Mikrotom (6) automatisch erfaßt und eine dieser zuzuordnende Kennzeichnung (1) auf mindestens einen Objektträger (2) automatisch übertragen wird und daß ausschließlich dieser mit der Kennzeichnung (1) versehene Objektträger (2) zu dem Zeitpunkt, an dem eine mikrotomierte Gewebeprobe auf einen Objektträger (2) gebracht werden muß, am Arbeitsplatz des Mikrotoms (6) für die manuelle Aufbringung der mikrotomierten Gewebeprobe der am Mikrotom (6) arbeitenden Fachkraft dargeboten wird.

2. Verfahren nach Anspruch 1,
**dadurch gekennzeichnet,**
**daß** mit jeder Herstellung einer mikrotomierten Gewebeprobe die Kennzeichnungsinformation (3) erfaßt und ein Objektträger (2) mit einer Kennzeichnung (1) hergestellt und dargeboten wird.

3. Verfahren nach Anspruch 1 oder 2,
**dadurch gekennzeichnet,**
**daß** die Kennzeichnung (1) des mindestens einen Objektträgers (2) der Kennzeichnungsinformation (3) entspricht.

4. Verfahren nach Anspruch 1, 2 oder 3,
**dadurch gekennzeichnet,**
**daß** die Kennzeichnungsinformation (3) neben der Herkunftszuordnung der Gewebeprobe (5) weitere Informationen enthält.

5. Verfahren nach einem der Ansprüche 1, 2 oder 4,
**dadurch gekennzeichnet,**
**daß** die Kennzeichnung (1) der Objektträger (2) aufgrund eines Inforrnationsverteilungsschlüssels neben der Herkunftszuordnung mit weiteren Informationen geladen wird, welche die mikrotomierte Gewebeprobe eines Objektträgers (2) individualisieren.

6. Verfahren nach Anspruch 5,
**dadurch gekennzeichnet,**
**daß** die weiteren Informationen Hearbeitungsanweisungen für die mikrotomierte Gewebeprobe des Objektträgers (2) enthalten.

7. Verfahren nach Anspruch 5 oder 6,
**dadurch gekennzeichnet,**
**daß** die weiteren Informationen Auswertungshinweise für die mikrotomierte Gewebeprobe des Objektträgers (2) sind.

8. Verfahren nach Anspruch 5, 6 oder 7,
**dadurch gekennzeichnet,**
**daß** der Informationsverarbeitungsschlüssel und die weiteren Informationen in der Kennzeichnungsinformation (3) enthalten sind.

9. Vorrichtung zur Durchführung eines Verfahrens nach einem der Ansprüche 1 bis 8,
**dadurch gekennzeichnet,**
**daß** sie eine Erfassungseinrichtung (7) zur Erfassung der Kennzeichnungsinformation (3) während der Träger (4) mit einer nicht mikrotomierten Gewebeprobe (5) in der Halteeinrichtung (8) eines Mikrotoms (6) angeordnet ist, aufweist, sowie eine Kennzeichnungseinrichtung (9) zur Ausstattung eines Objektträgers (2) mit einer Kennzeichnung (1), eine Übertragungseinrichtung (10) zur Informationsübertragung zwischen der Erfassungseinrichtung (7) und der Kennzeichnungseinrichtung (9) und eine am Arbeitsplatz des Mikrotoms (6) installierte Ausgabeeinrichtung (11) für die Entnahme von mit einer Kennzeichnung (1) versehenen Objektträgern (2) durch die am Mikrotom (6) arbeitende Fachkraft zum manuellen Aufbringen der mikrotomierten Gewebeprobe.

10. Vorrichtung nach Anspruch 9,
**dadurch gekennzeichnet,**
**daß** die Erfassungseinrichtung (7) derart ausgebildet ist, daß sie nach dem Positionieren eines Trägers (4) im Mikrotom (6) die Kennzeichnungsinformation (3) erfaßt, und daß sie die Kennzeichnung (1) mindestens eines Objektträgers (2) veranlaßt.

11. Vorrichtung nach Anspruch 9,
**dadurch gekennzeichnet,**
**daß** die Erfassungseinrichtung (7) derart ausgebildet ist, daß es bei der Herstellung einer mikrotomierten Gewebeprobe zu einer Erfassung der Kennzeichnungsinformation (3) und zu einem Befehl an die Kennzeichnungseinrichtung (9) zur Vornahme der Kennzeichnung (1) eines Objektträgers (2) kommt.

12. Vorrichtung nach Anspruch 9, 10 oder 11,
**dadurch gekennzeichnet,**
**daß** sich die Kennzeichnungsinformation (3) auf einem separaten Datenträger befindet, die einem Träger (4) mittels eines Identifikationscodes zuordenbar ist.

13. Vorrichtung nach Anspruch 9, 10 oder 11,
**dadurch gekennzeichnet,**
**daß** die Kennzeichnungsinformation (3) auf einem Informationsträger (15) geladen ist, der mit dem Träger (4) körperlich verbunden ist.

14. Vorrichtung nach einem der Ansprüche 9 bis 13,
**dadurch gekennzeichnet,**
**daß** die Ausgabeeinrichtung (11) derart ausgestaltet ist, daß sie die Darbietung eines mit einer Kennzeichnung (1) versehenen Objektträgers (2) abbricht, wenn ein neuer Träger (4) im Mikrotom (6) plaziert wird.

15. Vorrichtung nach einem der Ansprüche 9 bis 14,
**dadurch gekennzeichnet,**
**daß** sie eine Steuerung (12) zur Durchführung der Kennzeichnung (1) der Objektträger (2) aufweist.

16. Vorrichtung nach Anspruch 15,
**dadurch gekennzeichnet,**
**daß** sie eine Befehlseingabe (13, 13', 13") für die Bestimmung der Art und/oder der Anzahl der Kennzeichnungen (1) aufweist.

17. Vorrichtung nach Anspruch 9 bis 16,
**dadurch gekennzeichnet,**
**daß** sie ein Depot (16) für noch ungekennzeichnete Objektträger (2) aufweist.

18. Vorrichtung nach einem der Ansprüche 13 bis 17,
**dadurch gekennzeichnet,**
**daß** die Steuerung (12) derart ausgebildet ist, daß die Anzahl der mit einer Kennzeichnung (1) zu versehenden Objektträger (2) vorgebbar ist.

19. Vorrichtung nach Anspruch 18,
**dadurch gekennzeichnet,**
**daß** die Steuerung (12) derart ausgebildet ist, daß die Anzahl der mit einer Kennzeichnung (1) zu versehenden Objektträger (2) manuell erhöht werden kann.

20. Vorrichtung nach Anspruch 15 bis 19,
**dadurch gekennzeichnet,**
**daß** die Steuerung (12) derart ausgebildet ist, daß sie die Informationen für die Vornahme der Anzahl und/oder Art der Kennzeichnung (1) einer diese Information enthaltenden Kennzeichnungsinformation (3) entnimmt.

21. Vorrichtung nach Anspruch 9 bis 20,
**dadurch gekennzeichnet,**
**daß** sie eine Anzeigeeinrichtung (14) zur Darstellung von Informationen und/oder Arbeitsschritten aufweist.

22. Vorrichtung nach Anspruch 13 bis 21,
**dadurch gekennzeichnet,**
**daß** die Erfassungseinrichtung (7), die Träger (4) und die Halteeinrichtung (12) derart ausgebildet und angeordnet sind, daß die Erfassungseinrichtung (7) die Kennzeichnungsinformation (3) von einem Lesefeld (15') des Trägers (4) ablesen kann.

23. Vorrichtung nach Anspruch 22,
**dadurch gekennzeichnet,**
**daß** Träger (4) und Halteeinrichtung (11) derart ausgebildet und aufeinander abgestimmt sind, daß sich die Spannbacken (17) einer als Spanneinrichtung ausgebildeten Halteeinrichtung (11) außerhalb des Lesefeldes (15') eines eingespannten Trägers (4) befinden.

24. Vorrichtung nach einem der Ansprüche 9 bis 23,
**dadurch gekennzeichnet,**
**daß** eine Vorrichtung (18) zur Vorbereitung der Durchführung des Verfahrens vorgesehen ist, wobei der Träger (4) mit einem Informationsträger (15, 15') für die Zuordnung ausgestattet ist, und die Vorrichtung (18) eine Einrichtung (19) zur Erstellung der Kennzeichnungsinformation (3) aufweist, wobei diese Einrichtung (19) sowie der Informationsträger (15, 15') und ein dem Träger (4) zugeordneter Datenträger für die Erstellung einer Kennzeichnungsinformation (3) ausgebildet sind, die neben der Herkunftszuordnung der Gewebeprobe (5) einen Informationsverteilungsschlüssel und Informationen beinhalten, die einer individuellen Informationsausstattung der Kennzeichnung (1) von mehreren Objektträgern (2) mit mikrotomierten Gewebeproben dienen, die von derselben noch nicht mikrotomierten Gewebeprobe (5) stammen.

25. Vorrichtung nach Anspruch 24,
**dadurch gekennzeichnet,**
**daß** die Einrichtung (19) zur Erstellung der Kennzeichnungsinformation (3) derart ausgebildet ist, daß sie letztere auf einen separaten Datenträger lädt und diesen sowie den Informationsträger (15, 15') des Trägers (4) mit einem Identifikationscode zur gegenseitigen Zuordnung versieht.

26. Vorrichtung nach Anspruch 24,
**dadurch gekennzeichnet,**
**daß** die Einrichtung (19) und der Informationsträger (15, 15') derart ausgebildet, sind, daß letzterer mit der gesamten Kennzeichnungsinformation (3) ladbar ist.

27. Vorrichtung nach Anspruch 24, 25 oder 26
**dadurch gekennzeichnet,**
**daß** die Informationsausstattungen der Objektträger (2) Bearbeitungsanweisungen für die mikrotomierten Gewebeproben enthalten.

28. Vorrichtung nach einem der Anspruch 24 oder 27,
**dadurch gekennzeichnet,**
**daß** die informationsausstattungen der Objektträger (2) Auswertungsanweisungen für die mikrotomierten Gewebeproben enthalten.

29. Vorrichtung nach einem der Ansprüche 24 bis 28,
**dadurch gekennzeichnet,**
**daß** die Informationsausstattungen als maschinell erfaß- und umsetzbare Gerätesteuerungsanweisungen ausgestaltet sind.

## Claims

1. A method for assigning labels (1) of microscope slides (2) for microtomized tissue specimens yet to be placed thereon to labeling data (3) assigned to a carrier (4) for an associated tissue specimen (5) not yet microtomized,
**characterized in that**
said labeling data (3) pertaining to said carrier (4) are automatically acquired when said carrier (4) is placed in a microtome (6) and a label (1) to be assigned to said labeling data is automatically transferred to at least one microscope slide (2) and that precisely this microscope slide (2) provided with said label (1) is presented to the operator working at the microtome (6) station for manual transfer of the microtomized tissue specimen, this taking place at that point in time at which a microtomized tissue specimen is required to be transferred to said microscope slide (2).

2. The method as defined in claim 1,
**characterized in that**
with each production of a microtomized tissue specimen said labeling data (3) are acquired and a microscope slide (2) bearing a label (1) is produced and presented.

3. The method as defined in claim 1 or claim 2,
**characterized in that**
said label (1) of the at least one microscope slide (2) corresponds to the labeling data (3).

4. The method as defined in claim 1.2 or claim 3,
**characterized in that**
said labeling data (3) contain not only an indication of the origin of the tissue specimen (5) but also other information.

5. The method as defined in any one of claims 1, 2, or 4,
**characterized in that**
said label (1) on said microscope slide (2) is charged, by reason of an information distribution key, not only with the indication of origin but also with other information which individualizes the microtomized tissue specimen on a microscope slide (2).

6. The method as defined in claim 5,
**characterized in that**
said other information includes processing instructions for said microtomized tissue specimen on said microscope slide (2).

7. The method as defined in claim 5 or claim 6,
**characterized in that**
said other information consists of evaluation details concerning said microtomized tissue specimen on said microscope slide (2).

8. The method as defined in claim 5, claim 6, or claim 7,
**characterized in that**
said information processing key and said other information are included in said labeling data (3).

9. A device for carrying out a method as defined in any one of claims 1 to 8,
**characterized in that**
said device comprises a data entry device (7) for acquisition of said labeling data (3) when said carrier (4) carrying a non-microtomized tissue specimen (5) is placed in the holding device (8) of a microtome (6), and also a labeling device (9) for providing a microscope slide (2) with a label (1), a transmission system (10) for information transfer between said data entry device (7) and said labeling device (9) and output means (11) installed on the microtome (6) facilities for the withdrawal of microscope slides (2) that are provided with a label (1), said withdrawal being carried out by the operator working at the microtome (6) station for manual transfer of the microtomized tissue specimen.

10. The device as defined in claim 9,
**characterized in that**
said data entry device (7) is configured in such a manner that after a carrier (4) has been placed in the microtome (6) it acquires the labeling data (3) and causes the transfer of said label (1) to at least one microscope slide (2).

11. The device as defined in claim 9,
**characterized in that**
said data entry device (7) is configured in such a manner that when a microtomized tissue specimen is produced the labeling data (3) are acquired and a command is sent to said labeling device (9) for transferring a label (1) to a microscope slide (2).

12. The device as defined in claim 9, claim 10, or claim 11,
**characterized in that**
said labeling data (3) are stored on a separate data medium that can be assigned to a carrier (4) by means of an identification code.

13. The device as defined in claim 9, claim 10, or claim 11,
**characterized in that**
said labeling data (3) are stored on an information carrier (15) that is physically attached to said carrier (4).

14. The device as defined in any one of claims 9 to 13,
**characterized in that**
said output means (11) are configured in such a manner that they stop the presentation of a microscope slide (2) provided with a label (1) when a new carrier (4) is placed in said microtome (6).

15. The device as defined in any one of claims 9 to 14,
**characterized in that**
said device has control means (12) for transferring a label (1) to said microscope slide (2).

16. The device as defined in claim 15,
**characterized in that**
said device comprises command input means (13, 13', 13") for determination of the type and/or number of transfers of a label (1).

17. The device as defined in any one of claims 9 to 16,
**characterized in that**
it comprises a depot (16) for further unlabelled microscope slides (2).

18. The device as defined in any one of claims 13 to 17,
**characterized in that**
said control means (12) are configured in such a manner that the number of microscope slides (2) to be provided with a label (1) can be predefined.

19. The device as defined in claim 18,
**characterized in that**
said control means (12) are configured in such a manner that the number of microscope slides (2) to be provided with a label (1) can be manually increased.

20. The device as defined in any one of claims 15 to 19,
**characterized in that**
said control means (12) are configured in such a manner that they acquire the information for effecting the number and/or type of transfers of said label (1) from the labeling data (3) containing this information.

21. The device as defined in any one of claims 9 to 20,
**characterized in that**
it comprises a display device (14) for displaying information and/or method steps.

22. The device as defined in any one of claims 13 to 21,
**characterized in that**
said data entry device (7), said carrier (4), and said holding device (12) are configured and arranged in such a manner that said data entry device (7) can read off said labeling data (3) from a read-off area (15') on said carrier (4).

23. The device as defined in claim 22,
**characterized in that**
said carrier (4) and said holding device (11) are configured and interrelated in such a manner that gripping jaws (17) of a holding device (11) in the form of a clamping device are located outside the read-off area (15') of a clamped carrier (4).

24. The device as defined in any one of claims 9 to 23,
**characterized in that**
a device (18) for preparing the execution of the method is provided, in which said carrier (4) is equipped with an information carrier (15, 15') for effecting assignment and said device (18) has means (19) for creating said labeling data (3), and said means (19) and also said information carrier (15, 15') and a data medium assigned to said carrier (4) and designed to create labeling data (3) are configured in such a manner that said labeling data include not only the indication of origin of the tissue specimen (5) but also an information distribution key and information providing an individual information feature for the transfer of a label (1) to a plurality of microscope slides (2) carrying microtomized tissue specimens originating from the same non-microtomized tissue specimen (5).

25. The device as defined in claim 24,
**characterized in that**
said means (19) for creating said labeling data (3) are configured in such a manner that they store the latter on a separate data medium and provide this and said information carrier (15, 15') of said carrier (4) with an identification code for assignment to each other.

26. The device as defined in claim 24,
**characterized in that**
said means (19) and said information carrier (15, 15') are configured in such a manner that the latter can be charged with the entire labeling data (3).

27. The device as defined in claim 24, claim 25, or claim 26,
**characterized in that**
the information features of said microscope slides (2) contain processing instructions for the microtomized tissue specimens.

28. The device as defined in claim 24 or claim 27,
**characterized in that**
the information features of said microscope slides (2) contain evaluation instructions for the microtomized tissue specimens.

29. The device as defined in any one of claims 24 to 28,
**characterized in that**
the information features are in the form of device control instructions that are capable of being acquired and executed mechanically.

## Revendications

1. Procédé pour l'attribution du marquage (1) de porte-objets (2) pour des échantillons de tissu microtomés à appliquer pour une information de marquage (3), qui est associée à un support (4) d'un échantillon de tissu respectif non encore microtomé (5),
**caractérisé en ce que**
l'information de marquage (3) associée au support (4) est saisie automatiquement pendant la disposition de ce dernier dans le microtome et un marquage (1) devant être affecté est transmis automatiquement sur au moins un porte-objets (2) et **en ce qu'**exclusivement ce porte-objets (2) muni d'un marquage (1) doit être proposé au personnel qualifié travaillant sur le microtome (6) au moment où un échantillon microtomé doit être amené sur un porte-objets (2), au poste de travail du microtome (6) pour l'application manuelle de l'échantillon de tissu.

2. Procédé selon la revendication 1, **caractérisé en ce qu'**avec chaque fabrication d'un échantillon de tissu microtomé, l'information de marquage (3) est saisie et un porte-objet (2) est fabriqué et proposé avec un marquage (1).

3. Procédé selon la revendication 1 ou 2, **caractérisé en ce que** le marquage (1) d'au moins un porte-objet (2) correspond à l'information de marquage (3).

4. Procédé selon les revendications 1, 2 ou 3, **caractérisé en ce que** l'information de marquage (3) contient outre l'affectation d'origine de l'échantillon de tissu (5) d'autres informations.

5. Procédé selon l'une des revendications 1, 2 ou 4, **caractérisé en ce que** le marquage (1) des porte-objets (2) est chargé, en plus de l'affectation d'origine, d'autres informations sur la base d'une clé de répartition d'informations, qui individualisent l'échantillon de tissu microtomé d'un porte-objets (2).

6. Procédé selon la revendication 5, **caractérisé en ce que** les autres informations contiennent des instructions de traitement pour l'échantillon de tissu microtomé d'un porte-objets (2).

7. Procédé selon la revendication 5 ou 6, **caractérisé en ce que** les autres informations sont des instructions d'analyse pour l'échantillon de tissu microtomé d'un porte-objets (2).

8. Procédé selon la revendication 5, 6 ou 7,
**caractérisé en ce que** la clé de traitement d'information et les autres informations sont contenues dans l'information de marquage (3).

9. Dispositif pour la mise en oeuvre d'un procédé selon l'une des revendications 1 à 8, **caractérisé en ce que** qu'il présente un dispositif de saisie (7) pour la saisie de l'information de marquage (3), tandis que le support (4) avec un échantillon de tissu non microtomé (5) est disposé dans le dispositif de retenue (8) d'un microtome (6), ainsi qu'un dispositif de marquage (9) doter d'un porte-objets (2) d'un marquage (1), un dispositif de transmission (10) pour la transmission d'informations entre le dispositif de saisie (7) et le dispositif de marquage (9) et un dispositif de distribution (11) installé sur le poste de travail du microtome (6), pour le prélèvement de porte-objets (2) munis d'un marquage (1) par le personnel spécialisé travaillant sur le microtome (6) pour l'application manuelle de l'échantillon de tissu microtomé.

10. Dispositif selon la revendication 9, **caractérisé en ce que** le dispositif de saisie (7) est réalisé de sorte qu'il saisit après le positionnement d'un support (4) dans le microtome (6), l'information de marquage (3) et **en ce qu'**il occasionne le marquage (1) d'au moins un porte-objet (2).

11. Dispositif selon la revendication 9, **caractérisé en ce que** le dispositif de saisie (7) est conçu de sorte que lors de la fabrication d'un échantillon de tissu microtomé, il se produit une saisie de l'information de marquage (3) et une instruction au dispositif de marquage (9) pour effectuer le marquage (1) d'un porte-objet (2).

12. Dispositif selon la revendication 9, 10 ou 11, **caractérisé en ce que** l'information de marquage (3) se trouve sur un support de données éparé qui peut être associé à un support (4) au moyen d'un code d'identification.

13. Dispositif selon la revendication 9, 10 ou 11, **caractérisé en ce que** l'information de marquage (3) est chargée sur un support d'information (15) qui est relié corporellement au support (4).

14. Dispositif selon l'un des revendications 9 à 13, **caractérisé en ce que** le dispositif de sortie (11) est réalisé de manière à interrompre le placement d'un porte-objet (2) muni d'un marquage (1) si un nouveau support (4) doit être placé dans le microtome (6).

15. Dispositif selon l'une des revendications 9 à 14, **caractérisé en ce qu'**il présente une commande (12) pour la mise en oeuvre du marquage (1) des porte-objets (2).

16. Dispositif selon la revendication 15, **caractérisé en ce que** qu'il présente une saisie d'instruction (13, 13', 13") pour la détermination de la nature et/ou du nombre des marquages (1).

17. Dispositif selon la revendication 9 à 16, **caractérisé en ce qu'**il présente un dépôt (16) pour des porte-objets (2) non encore marqué.

18. Dispositif selon l'une des revendications 13 à 17, **caractérisé en ce que** la commande (12) est conçue de sorte que le nombre des porte-objets (2) à doter d'un marquage (1) peut être prédéterminé.

19. Dispositif selon la revendication 18, **caractérisé en ce que** la commande (12) est réalisée de sorte que le nombre des porte-objets (2) à doter d'un marquage (1) peut être augmenté manuellement.

20. Dispositif selon la revendication 15 à 19,
**caractérisé en ce que** la commande (12) est réalisée de sorte qu'elle prélève à partir information de marquage (3) contenant cette information, les informations pour effectuer le nombre et/ou la nature du marquage (1).

21. Dispositif selon la revendication 9 à 20, **caractérisé en ce que** qu'il présente un dispositif d'affichage (14) pour la représentation d'informations et/ou d'étapes de travail.

22. Dispositif selon la revendication 13 à 21, **caractérisé en ce que** le dispositif de saisie (7), les supports (4) et le dispositif de retenue (12) sont réalisés et disposés de manière que le dispositif de saisie (7) puisse lire les informations de marquage (3) par un champ de lecture (15') du support (4).

23. Dispositif selon la revendication 22, **caractérisé en ce que** des supports (4) et un dispositif de retenue (11) sont réalisés de manière correspondante entre eux de sorte que les mâchoires de serrage (17) d'un dispositif de retenue (11) réalisé comme dispositif de serrage se trouvent à l'extérieur du champ de lecture (15') d'un support serré (4).

24. Dispositif selon l'une des revendications 9 à 23, **caractérisé en ce qu'**il est prévu un dispositif (18) pour la préparation de la mise en oeuvre du procédé, le support (4) étant muni d'un support d'information (15, 15') pour l'affectation, et le dispositif (18) présentant un dispositif (19) pour la détermination de l'information de marquage (3), ce dispositif (19) ainsi que le support d'information (15 15') et un support de données associé au support (4) sont réalisés pour la détermination d'une information de marquage (3) qui contient, outre l'affectation d'origine de l'échantillon de tissu (5), une clé de répartition d'information et des informations qui servent à l'affectation individuelle d'une information du marquage (1) de plusieurs porte-objets (2) avec des échantillons de tissu microtomés qui proviennent du même échantillon de tissu non encore microtomé (5).

25. Dispositif selon la revendication 24, **caractérisé en ce que** le dispositif (19) est conçu pour la réalisation de l'information de marquage de sorte qu'il charge cette dernière sur un support de données séparés et dote ce dernier ainsi que le support d'information (15, 15') du support (4) d'un code d'identification pour une attribution réciproque.

26. Dispositif selon la revendication 24, **caractérisé en ce que** le dispositif (19) et le support d'information (15, 15') sont conçus de sorte que ce dernier peut être chargé de l'ensemble de l'information de marquage (3).

27. Dispositif selon la revendication 24, 25 ou 26,
**caractérisé en ce que** les dotations d'information des porte-objets (2) contiennent des instructions de traitement pour les échantillons de tissu microtomés.

28. Dispositif selon l'une des revendications 24 ou 27,
**caractérisé en ce que** les dotations d'informations des porte-objets (2) contiennent des instructions d'analyse pour les échantillons de tissu microtomés.

29. Dispositif selon l'une des revendications 24 à 28,
**caractérisé en ce que** les dotations d'informations sont réalisées comme des instructions de commande d'appareil saisissables et exécutables.
